(19) Europäisches Patentamt
European Patent Office
Office européen des brevets

(11) Veröffentlichungsnummer: **0 395 977 A1**

(12) **EUROPÄISCHE PATENTANMELDUNG**

(21) Anmeldenummer: **90107670.3**

(51) Int. Cl.5: **C07D 239/36**

(22) Anmeldetag: **23.04.90**

(30) Priorität: **24.04.89 CH 1542/89**
**30.11.89 CH 4289/89**

(43) Veröffentlichungstag der Anmeldung:
**07.11.90 Patentblatt 90/45**

(84) Benannte Vertragsstaaten:
**AT BE CH DE DK ES FR GB IT LI LU NL SE**

(71) Anmelder: **LONZA AG**
**Gampel/Wallis Geschäftsleitung Basel**
**CH-4002 Basel(CH)**

(72) Erfinder: **Mills, Lester Stephen, Dr.**
**Furkastrasse 65**
**Naters/Kanton Wallis(CH)**
Erfinder: **Previdoli, Felix, Dr.**
**Rhonesandstrasse 24**
**Brig/Kanton Wallis(CH)**

(74) Vertreter: **Weinhold, Peter, Dr. et al**
**Patentanwälte Dr. V. Schmied-Kowarzik**
**Dipl.-Ing. G. Dannenberg Dr. P. Weinhold Dr.**
**D. Gudel Dipl.-Ing. S. Schubert Dr. P. Barz**
**Siegfriedstrasse 8**
**D-8000 München 40(DE)**

(54) **Verfahren zur Herstellung von 4-Hydroxy-6-polyfluor-alkylpyrimidinen.**

(57) Beschrieben wird ein Verfahren zur Herstellung von 4-Hydroxy-6-polyfluoralkylpyrimidinen, ausgehend von einem Polyfluoralkylcarbonylessigsäureniedrigalkylester und einem Formamidinsalz, sowie ein neues Zwischenprodukt, das 4-Hydroxy-6-pentafluorehtylpyrimidin.
Diese 4-Hydroxy-6-polyfluoralkylpyrimidine können zur Herstellung von Trifluormethylpyrimidinen, Trifluormethylpurinen und Pentafluorethylpyrimidinen verwendet werden.

EP 0 395 977 A1

**Verfahren zur Herstellung von 4-Hydroxy-6-polyfluoralkylpyrimidenen**

Die Erfindung betrifft ein Verfahren zur Herstellung von 4-Hydroxy-6-polyfluoralkylpyrimidinen der alllgemeinen Formel 1

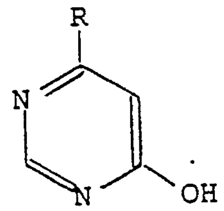

(1)

sowie ein neues Pyrimidin, das 4-Hydroxy-6-pentafluorethylpyrimidin.

Das 4-Hydroxy-6-trifluormethylpyrimidin ist ein wertvolles Zwischenprodukt zur Herstellung von Trifluormethylpyrimidinen und Trifluormethylpurinen. 4-Hydroxy-6-pentafluorethylpyrimidin ist aus der Literatur nicht bekannt, stellt jedoch ein wichtiges Zwischenprodukt in der Herstellung von Pentafluorethylpyrimidinen dar.

Ein Verfahren zur Herstellung von 4-Hydroxy-6-trifluormethylpyrimidin aus 4-Hydroxy-2-mercapto-6-trifluormethylpyrimidin, ausgehend von Trifluoracetessigsäureethylester, ist im J.Am. Chem.Soc., Vol. 80, 1958, S.5744-5752 beschrieben.

In der hier beschriebenen Herstellung wird Thioharnstoff und Trifluoracetessisäureethylester zu 4-Hydroxy-2-mercapto-6-trifluormethylpyrimidin umgesetzt, welches anschliessend in einer weiteren Stufe in Gegenwart von Wasserstoff und Raney-Nickel in das Produkt überführt wird. Dieses 2-Stufen-Verfahren führt nur zu einer Gesamtausbeute von 48,4%, bezogen auf eingesetzten Trifluoracetessisäureethylester.

Ein Nachteil dieses Verfahrens besteht darin, dass aus dem Zwischenprodukt 4-Hydroxy-2-mercapto-6-trifluormethylpyrimidin die SH-Gruppe entfernt werden muss und das dabei anfallende Produkt verunreinigt ist.

Die Aufgabe der vorliegenden Erfindung bestand darin, ein ökonomischeres Verfahren zur Herstellung von 4-Hydroxy-6-polyfluoralkylpyrimidinen zur Verfügung zu stellen, das sich durch hohe Ausbeuten und Reinheit auszeichnet sowie ein neues Zwischenprodukt zur Herstellung von Pentafluorethylpyrimidinen zur Verfügung zu stellen. Erreicht wird dies durch ein Verfahren gemäss dem Patentanspruch 1.

Das Verfahren zur Herstellung von 4-Hydroxy-6-polyfluoralkylpyrimidinen der allgemeinen Formel 1

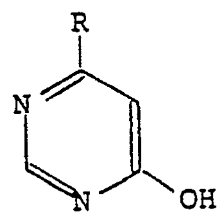

(1)

in welcher R eine Trifluormethyl- oder Pentafluorethylgruppe bedeutet, kann durch Zusammenmischen eines Polyfluoralkylcarbonylessigsäureniedrigalkylesters der allgemeinen Formel 2

$R - \overset{\overset{O}{\|}}{C} - CH_2 - COOR_1$ (2)

in welcher R die oben genannte Bedeutung hat und $R_1$ eine Niederalkylgruppe mit 1 bis 4 C-Atomen bedeutet, mit einem Formamidinsalz der allgemeinen Formel 3

$H\underset{\underset{NH}{\|}}{C} - NH_2 \cdot HX$ (3)

in welcher X Acetat oder Chlorid bedeuten kann, entweder in Gegenwart eines Alkalialkoholats oder in Gegenwart von Ammoniumcarbonat oder eines Alkalicarbonats durchgeführt werden.

Als Polyfluoralkylcarbonylessigsäureniedrigalkylester der allgemeinen Formel 2 kann ein Polyfluoralkylcarbonylessigsäure-methyl-, -ethyl-, -propyl- und -butyl-ester verwendet werden, vorzugsweise wird 4,4,5,5,5-Pentafluor-3-oxopentansäureethylester oder Trifluoracetessigsäureethylester verwendet.

Als Formamidinsalz kann Formamidinacetat oder Formamidinhydrochlorid angewendet werden, vorzugsweise wird Formamidinacetat verwendet.

Als Alkalialkoholate können Natrium- und Kaliumalkoholate verwendet werden, vorzugsweise wird Natriummethylat verwendet.

Als Alkalicarbonat kann Natrium- und Kaliumcarbonat verwendet werden, vorzugsweise wird Natriumcarbonat verwendet.

Die Umsetzung des Polyfluoralkylcarbonylessigsäureniedrigalkylesters kann mit 1,0 bis 2,0 Aequivalenten Formamidinsalz durchgeführt werden, vorzugsweise wird die Reaktion von 4,4,5,5,5-Pentafluor-3-oxopentansäureethylester mit 1,0 bis 1,5 Aequivalenten Formamidinsalz, vorzugsweise mit 1,1 bis 1,3 Aequivalenten, durchgeführt.

Die Reaktion von Trifluoracetessigsäurethylester wird mit 1,3 bis 2,0 Aequivalenten Formamidinsalz, vorzugsweise mit 1,4 bis 1,5 Aequivalenten, durchgeführt.

Als Lösungsmittel kann entweder ein niedrig siedender aliphatischer Alkohol oder eine Mischung aus einem niedrig siedenden aliphatischen Alkohol und einem Aromaten angewendet werden. Als aliphatischer Alkohol können Methanol, Ethanol, Propanol oder Butanol angewendet werden.

Als Aromat kann Toluol, Benzol und Xylol angewendet werden, zweckmässig kommt Toluol zur Anwendung. Vorzugsweise wird die Umsetzung von 4,4,5,5,5-Pentafluor-3-oxopentansäureethylester in einem Lösungsmittelgemisch aus Methanol und Toluol durchgeführt.

Die Umsetzung des 4,4,5,5,5-Pentafluor-3-oxopentansäureethylesters wird bei Temperaturen von 40° C bis Rückflusstemperatur, vorzugsweise bei Rückflusstemperatur, unter Rühren, in einer Reaktionszeit von 1 bis 24 Stunden, vorzugsweise 2 bis 5 Stunden, durchgeführt.

Anschliessend wird der pH-Wert auf einen Bereich von 3 bis 5 eingestellt, vorzugsweise auf pH 3,5 bis 4,5.

Die Umsetzung von Trifluoracetessisäureethylester wird vorzugsweise in Methanol durchgeführt.

Die Umsetzung des Trifluoracetessigsäureethylesters wird bei Temperaturen von 40 bis 65° C, vorzugsweise bei 60 bis 65° C, unter Rühren, in einer Reaktionszeit von 5 bis 24 Stunden, vorzugsweise von 5,5 Stunden.

Anschliessend wird der pH-Wert auf einen Bereich von 2,0 bis 4,0 eingestellt, vorzugsweise auf pH 2,5 bis 3,5.

Als Säuren können nichtoxidierende Mineralsäuren, wie beispielsweise Salzsäure, Schwefelsäure und Phosphorsäure angewendet werden, vorzugsweise wird Salzsäure angewendet. Im bekannten Stand der Technik wird für das 4-Hydroxy-6-trifluormethylpyrimidin ein Schmelzpunkt von 160 bis 162° C angegeben. Es muss daher angenommen werden, dass das erfindungsgemäss hergestellte Produkt gegenüber dem Produkt aus dem Stand der Technik eine wesentlich höhere Reinheit aufweist.

4-Hydroxy-6-pentafluorethylpyrimidin und 4-Hydroxy-6-trifluormethylpyrimidin können in an sich bekannter und für das Trifluormethyl-Derivat in J. Am. Chem. Soc., Vol. 80, 1958, S. 5744-5752 beschriebenen Weise zur Synthese weiterer Pyrimidinderivate verwendet werden bzw. auch zur Synthese von Purinen.

## Beispiel 1

### 4-Hydroxy-6-pentafluorethylpyrimidin

5,2 g 4,4,5,5,5-Pentafluor-3-oxopentansäureethylester (0,020 Mol), 2,3 g Formamidinacetat (0,022 Mol), 1,2 g Natriummethylat (0,022 Mol), Methanol (2,8 g) und Toluol (50 g) werden zusammen gemischt. Das Reaktionsgemisch wurde bis zum Rückfluss erhitzt und das Methanol, das Wasser und ein Teil des Toluols wurden mittels einem Kühler und Wasserabscheider während 2 Stunden abdestilliert. Weitere 1,2 g (0,022 Mol) Natriummethylat und Methanol (2,8 g) wurden zugegeben und nach 2 Stunden 20 Minuten wurde das Reaktionsgemisch auf Raumtemperatur abgekühlt, mit Wasser verdünnt und auf pH 4,5 mit 10%iger Salzsäure gestellt. Die wässrige Lösung wurde mit Ethylacetat extrahiert und die organische Phase abgetrennt, getrocknet ($Na_2SO_4$) und eingeengt.

Das Rohprodukt wurde mittels Kugelrohrdestillation gereinigt. Man erhielt 2,5 g schwachgelbes kristallines Produkt entsprechend einer Ausbeute von 48% bezogen auf eingesetzten 4,4,5,5,5-Pentafluor-3-oxopentansäureethylester.

$^1H$ NMR ($CDCl_3$, 300 MHz): $\delta$ 6,91 (1H, s)
8,42 (1H, s)
13,22 (1H, bs)

## Beispiel 2

4-Hydroxy-6-trifluormethylpyrimidin

Ein Gemisch von 435,0 g (2,3 Mol) Trifluoracetessigsäureethylester, 359,0 g (3,45 Mol) Formamidinacetat und 304 g (2,83 Mol) Natriumcarbonat in 800 g Methanol wurde unter Rühren auf ca. 60° C erhitzt. Nach einer Reaktionszeit von 5,5 Stunden wurde auf Raumtemperatur abgekühlt und mit 689 g (6,9 Mol) konzentrierter Salzsäure ein pH-Wert von 3,0 eingestellt. Das Methanol wurde mit Vakuum teilweise abdestilliert und der Rückstand mit 300 ml Wasser aufgeschlämmt. Nach Abkühlen auf 5 bis 10° C wurde das Rohprodukt abfiltriert, zweimal mit 200 ml kaltem Wasser gewaschen und bei 60° C bei einem Druck von 13332,2 Pa getrocknet.
Man erhielt 321,1 g weisses kristallines Produkt, entsprechend einer Ausbeute von 85%, bezogen auf eingesetzten Trifluoracetessigsäureethylester.
Smp.: 164 bis 165° C
$^1$H NMR: (DMSO-$d^6$, 300 MHz) $\delta$ 6,89 (s, 1H)
8,40 (s, 1H)
13,18 (bs, 1H)
MS: $M^+$ 164
IR (KBr) $cm^{-1}$ 3417, 3348, 3286, 3165, 3120, 3082, 3048, 3001, 2932, 2892, 2707, 2684, 2657, 1823, 1687, 1618, 1558, 1482, 1438, 1348, 1275, 1205
UV (MeOH) nm: 219, 279

**Ansprüche**

1. Verfahren zur Herstellung von 4-Hydroxy-6-polyfluoralkylpyrimidinen der allgemeinen Formel

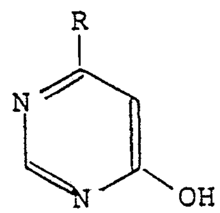

(1)

in welcher R eine Trifluormethyl- oder Pentafluorethylgruppe darstellt, dadurch gekennzeichnet, dass man einen Polyfluoralkylcarbonylessigsäureniedrigalkylester der allgemeinen Formel

$$R - \overset{O}{\overset{\parallel}{C}} - CH_2 - COOR_1 \quad (2)$$

in welcher R die oben genannte Bedeutung hat und $R_1$ eine Niederalkylgruppe mit 1 bis 4 C-Atomen bedeutet, mit einem Formamidinsalz der allgemeinen Formel

$$H\underset{\underset{NH}{\parallel}}{C} - NH_2 \cdot HX \quad (3)$$

in welcher X Acetat oder Chlorid bedeuten kann, in Gegenwart entweder eines Alkalialkoholats oder in Gegenwart von Ammoniumcarbonat oder eines Alkalicarbonats, vermischt und zum Endprodukt umsetzt.

2. Verfahren nach Patentanspruch 1, dadurch gekennzeichnet, dass man die Umsetzung entweder in einem niedrigsiedenden aliphatischen Alkohol oder in einer Mischung aus einem niedrigsiedenden aliphatischen Alkohol und einem Aromaten durchführt.

3. Verfahren nach mindestens einem der Ansprüche 1 und 2, dadurch gekennzeichnet, dass man als niedrigsiedenden aliphatischen Alkohol Methanol und als Aromaten Toluol verwendet.

4. Verfahren nach mindestens einem der Ansprüche 1 bis 3, dadurch gekennzeichnet, dass man als Alkalialkoholat Natriummethylat und als Alkalicarbonat Natriumcarbonat verwendet.

5. Verfahren nach mindestens einem der Ansprüche 1 bis 4, dadurch gekennzeichnet, dass man die Umsetzung von 40° C bis Rückflusstemperatur durchführt.

6. Verfahren nach mindestens einem der Ansprüche 1 bis 5, dadurch gegekennzeichnet, dass man die Umsetzung mit 1,0 bis 2,0 Aequivalenten Formamidinsalz durchführt.

7. Verfahren nach mindestens einem der Ansprüche 1 bis 6, dadurch gegekennzeichnet, dass, wenn man als Polyfluoralkylcarbonylessigsäureniedrigalkylester 4,4,5,5,5-Pentafluor-3-oxopentan säureethylester verwendet, die Umsetzung mit 1,0 bis 1,5 Aequivalenten Formamidinsalz in Gegenwart eines Alkalimethylats, gelöst in einer Mischung aus Methanol und Toluol, bei Temperaturen von 40° C bis Rückflusstemperatur durchführt.

8. Verfahren nach mindestens einem der Ansprüche 1 bis 6, dadurch gekennzeichnet, dass, wenn man als Polyfluoroalkylcarbonylesigsäureniedrigalkylester Trifluoracetessigsäureethylester verwendet, die Umsetzung mit 1,3 bis 2,0 Aequivalenten Formamidinsalz in Gegenwart eines Alkalicarbonats, gelöst in Methanol, bei Temperaturen von 40 bis 65 °C durchführt.

9. 4-Hydroxy-6-pentafluorethylpyrimidin.

10. Verwendung des 4-Hydroxy-6-trifluormethylpyrimidins zur Herstellung von Trifluormethylpyrimidinen und Trifluormethylpurinen.

11. Verwendung des 4-Hydroxy-6-pentafluorethylpyrimidins zur Herstellung von Pentafluorethylpyrimidinen.

Europäisches Patentamt

# EUROPÄISCHER RECHERCHENBERICHT

Nummer der Anmeldung

EP 90 10 7670

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | DE-A-2 120 247 (LONZA AG)<br>* ganzes Dokument *<br>--- | 1-4,6 | C 07 D 239/36 |
| A,D | JOURNAL OF THE AMERICAN CHEMICAL SOCIETY<br>Band 80, Nr. 21, 14. November 1958, Seiten 5744-5752; A. GINER-SOROLLA et al.: "Fluorine-containing pyrimidines and purines: Synthesis and Properties of Trifluoromethyl Pyrimidines and Purines" * Seiten 5746,5747 *<br>--- | 10,1 | |
| P,X | EP-A-0 338 686 (IMPERIAL CHEMICAL INDUSTRIES PLC)<br>* Seite 9, Herstellungsverfahren 2 *<br>----- | 9 | |

| RECHERCHIERTE SACHGEBIETE (Int. Cl.5) |
|---|
| C 07 D 239/00 |

Der vorliegende Recherchenbericht wurde für alle Patentansprüche erstellt

| Recherchenort | Abschlußdatum der Recherche | Prüfer |
|---|---|---|
| BERLIN | 01-08-1990 | VAN AMSTERDAM L.J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)